# EUROPEAN PATENT SPECIFICATION

(11) **EP 4 345 222 B1**
(45) Date of publication and mention of the grant of the patent: **07.01.2026**
(21) Application number: 22198589.8
(22) Date of filing: 29.09.2022
(51) Int. Cl.: E03D 9/00, A61B 5/00, A61B 5/20, A61B 5/282, A61B 5/053, A61B 5/024, A61B 5/1172, A61B 5/1171

(54) **SYSTEM AND METHOD FOR BOWEL MOVEMENT DETECTION**
SYSTEM UND VERFAHREN ZUR ERKENNUNG VON DARMBEWEGUNGEN
SYSTÈME ET PROCÉDÉ DE DÉTECTION DE MOUVEMENT INTESTINAL

(43) Date of publication of application: 03.04.2024
(73) Proprietor: Stichting IMEC Nederland, 6708 WH Wageningen (NL)
(72) Inventor: Wentink, Eva, 5591 MT Heeze (NL); Tourolle, Duncan, 6862 AV Oosterbeek (NL); Bax-Witteveen, Jolanda, 6021 HL Budel (NL); van Kraaij, Alex, 6532 CS Nijmegen (NL); Verbiest, Vera, 6721 SV Bennekom (NL)
(74) Representative: Körfer, Thomas

(56) References cited:
- EP-A1- 3 906 844
- CN-A- 110 693 455
- US-A1- 2016 374 619
- US-A1- 2018 078 191
- US-A1- 2022 074 918

## Description

The invention relates to noninvasive measurement and/or acquisition of physiological parameters, especially for detecting a bowel movement and/or urination, and for characterizing excrement or faeces in terms of a firmness level.

Generally, human excreta is a common source of information that may provide insight into the person's health. Particularly, an obstipation may be considered as a sign of an underlying or chronic illness, and a detection of obstipation may assist with diagnosis of said illness. Moreover, a detection of obstipation is relevant in settings where the person has difficulty in conveying the situation.

At present, diagnosis of obstipation relies on self-reporting from the person, which may lead to blood tests and/or further physical examinations. In most cases, the investigation of the excrement requires the harvesting of a sample, either a physical sample or a digital sample, e.g. via a camera, and the processing of the sample generally requires extensive technical expertise. Moreover, the use of a camera may be considered as an invasion of privacy.

For example, the document US 2020/0100725 A1 discloses a toilet system for health diagnosis that performs visual recordings of excrement, e.g. via a camera or a video camera, for collecting data, e.g. color, volume, or consistency, of the excrement produced by bowel movements of an individual. Further toilet systems are known from US 2022/074918 A1, US 2016/374619 A1, US 2018/078191 A1, EP 3906844 A1 and CN 110693455 A.

Accordingly, an object of the invention is to provide a system, a smart toilet and a method for bowel movement detection that address the aforementioned limitations in order to facilitate noninvasive and unobtrusive detection and monitoring of bowel movement health.

The object is solved by the features of the first independent claim for the system, the claims for the smart toilet and by the features of the second independent claim for the method. The dependent claims contain further developments. The invention is defined by the appended claims.

According to a first aspect of the disclosure, a system for bowel movement detection is provided. The system comprises a sensor module configured to generate one or more physiological parameter measurement data of a user and a timestamp associated with the one or more physiological parameter measurement data. The system further comprises a signal processing module configured to receive the one or more physiological parameter measurement data and further to correlate the one or more physiological parameter measurement data using the associated timestamp in order to detect at least one bowel movement segment and/or an urination process of the user.

In this regard, the one or more physiological parameter measurement data comprise at least one electrocardiogram (ECG) measurement data and at least one bio-impedance (BioZ) measurement data of the user. The signal processing module may perform further processing of the measurement data before or after the correlation. The further processing of the measurement data may comprise pre-processing such as filtering, motion artefact removal and signal quality assessment, time-domain analysis, frequency-domain analysis, linear regression, physiological parameter estimation, and the like.

The timestamp associated to the one or more physiological parameter measurement data may comprise a sequence of characters or encoded information that may identify a measurement session or event or instant. Preferably, the timestamp may comprise digital information regarding a date and/or a time, especially accurate to a small fraction of a second, attached to the digital measured data.

For example, a single or a string of data corresponding to ECG measurements and a single or a string of data corresponding to BioZ measurements may be correlated using the associated timestamp if the measurements are performed simultaneously in one measurement session or event or instant, since the timestamp associated to the measurements may depict identical or similar date and/or time information when the measurements are being carried out.

Therefore, the disclosure proposes an unobtrusive technique for detecting bowel movements and/or urination. Especially, the invention facilitates noninvasive acquisition of different physiological signals, such as but not limited to blood oxygen saturation, heart rate, respiration and cardiac output, blood pressure, temperature, and bio-impedance. Advantageously, a fusion and/or a correlation of the different physiological data can be performed in order to determine a bowel movement, e.g. via estimating stress or strain or lack thereof from the fused or correlated data during a bowel movement.

Further advantageously, the disclosure proposes a noninvasive detection of an urination process, and further to differentiate between a bowel movement process and an urination process of the user based on the different physiological signals, e.g. via detecting a change in measured bio-impedance due to a change in urine volume in bladder.

According to the invention, the signal processing module is further configured to compare the correlation of the one or more physiological parameter measurement data with a reference correlation of the one or more physiological parameter measurement data corresponding to a Bristol stool scale, and further to determine a firmness level of excrement during the at least one bowel movement segment.

For instance, the different levels of the Bristol stool chart may be categorized using previously collected physiological data and/or reference physiological data and/or correlations with annotated recordings and/or patterns of the physiological signals, thereby forming a reference database, which can be used for the comparison and/or for the firmness level detection of the excrement during the bowel movement.

In this regard, a reference correlation of ECG data and BioZ data may be generated based on the different levels, i.e. stool firmness levels, of the Bristol stool chart. For example, a reference correlation of the ECG data and BioZ data may be generated for a bowel movement resulting in faeces in form of separate hard lumps (severe constipation), faeces in form of a smooth sausage (normal), or faeces with liquid consistency (diarrhea). Afterwards, the measured ECG data and the measured BioZ data may be correlated and the correlated data obtained therefrom may be compared with the reference correlation.

Moreover, the reference database, especially the reference correlation, can be generated and/or stored correspond to a specific user of the system. Advantageously, a firmness level of the excrement can be determined or classified without any visual assessment of the excrement.

Preferably, the sensor module comprises at least two source electrodes configured to excite from at least two different measurement locations on the user, and at least two receiver electrodes configured to respectively measure response from the at least two different measurement locations, especially to generate the at least one ECG measurement data and/or the at least one BioZ measurement data. Advantageously, a tetrapolar or four-electrode measurement technique is facilitated which improves the measurement quality as well as the sensitivity and accuracy of the measured signals.

Preferably, the at least two source electrodes and/or the at least two receiver electrodes are multiplexed based on a time division and/or a frequency division multiplexing technique. For instance, a frequency division can be implemented for parallel acquisition of the ECG data (low frequency) and the BioZ data (high frequency), which facilitates a high flexibility in terms of measurement and performance.

Preferably, the one or more physiological parameter measurement data further comprise at least one weight measurement data. In this regard, the signal processing module is further configured to determine a mass of excrement based on the at least one weight measurement data during the at least one bowel movement segment. Additionally or alternatively, the signal processing module is further configured to determine a volume of urine based on the at least one weight measurement data during the urination process.

For instance, a bowel movement or an urination process may result in reduction of weight of a user, especially noticeable during the urination process or in severe diarrhea condition due to loss of body water. The weight measurement data may comprise body weight measurements of the user, for instance, consecutively measured at different times during the measurement session or event or instant, especially including body weight measurements before and after the bowel movement and/or the urination process. Advantageously, the weight measurement data can be effectively used to determine or estimate the mass of excrement when a bowel movement is detected. An average mass of the faeces weights about 0.11 kg (a quarter pound) to 0.45 kg (one pound), which can be detected form the weight measurement data effectively. Additionally, the weight measurement data can be effectively used to determine or estimate the amount or volume of urine produced, especially when no bowel movement is detected.

Preferably, the one or more physiological parameter measurement data further comprise at least one photoplethysmography (PPG) measurement data. In this regard, the sensor module comprises at least one further electrode configured to excite and/or measure response from at least one further measurement location on the user, especially to generate the at least one PPG measurement data.

The PPG measurement data may correspond to the perfusion of blood to the dermis and subcutaneous tissue of the skin, especially during each cardiac cycle, where each cardiac cycle appears as a peak. The PPG electrode may illuminate the skin with a light and may accordingly measure the amount of light either transmitted or reflected.

Advantageously, an effective correlation can be performed to determine bowel movements, e.g. by means of the assessment of heart rate and blood pressure especially during a bowel movement segment.

Preferably, the system further comprises a user detection module, e.g. comprising a biometric sensor, whereby the signal processing module is configured to obtain a user detection signal comprising at least one user parameter from the user detection module. In this regard, the at least one user parameter comprises one or more biometric attributes of the user including fingerprints, facial images, iris, and voice.

Additionally or alternatively, the user detection module is configured to detect the user based on the one or more physiological parameter measurement data. For example, the user detection module may perform an ECG-based human identification by extracting ECG traits or features from measured ECG signals and comparing the extracted ECG traits or features with reference ECG signals, e.g. previously measured and stored ECG signals, thereby generating the user detection signal.

For example, the system may comprise a sleep/wake-up control module configured to turn on or turn off the sensor module at least based on the user detection signal. The sleep/wake-up control module can be implemented as a separate module or can be implemented within the user detection module, which may additionally generate a sleep/wake-up signal to turn on or turn off the sensor module. Thus, a sleep/wake-up function for the sensor module can be enabled, which may advantageously increase the lifespan of the embedded sensors.

Preferably, the signal processing module is configured to generate measurement sessions based on the timestamp associated with the one or more physiological parameter measurement data, and further to assign the measurement sessions to the user detection signal and/or to the user. In this regard, a measurement session may include the initiation of the measurements, e.g. initiating the sensors, measurement data acquisition from the sensors, measurement data transmission to external peripherals, and the processing of the measurement data. Advantageously, user-specific measurement and/or detection can be performed.

Preferably, the signal processing module is configured to assess a signal quality of the one or more physiological parameter measurement data with respect to a pre-defined threshold and further to correlate the one or more physiological parameter measurement data based on the assessed signal quality.

In this regard, the quality of the detected signal can be determined based on one or more signal traits, such as but not limited to signal-to-noise ratio (SNR), peak-to-peak interval, and amplitude, of the detected signal during the physiological parameter measurements. Additionally or alternatively, the quality of the detected signal can be determined based on the known signal quality indices for physiological parameters including but not limited to perfusion, kurtosis, and skewness.

Preferably, the signal processing module may be configured to generate an alert signal based on the quality of the measurement data. In this regard, the alert signal may comprise a tone and/or a text notification and/or a voice announcement. For instance, the signal processing module may detect a linear translation in the positioning of the user based on the quality of the measurement data, and may notify the user via the alert signal whether the user positioning is needed to be amended. Advantageously, a feedback to the user regarding any anomalies during the measurement is also incorporated.

Preferably, the system further comprises a communication module comprising at least one of a ZigBee module, a Bluetooth module, a Wi-Fi module, a GPRS module, or an NB-IoT module, whereby the communication module is configured to transmit the one or more physiological parameter measurement data wirelessly.

Additionally or alternatively, the signal processing module further comprises at least one of a ZigBee module, a Bluetooth module, a Wi-Fi module, a GPRS module, or an NB-IoT module, whereby the signal processing module is further configured to receive the one or more physiological parameter measurement data wirelessly, especially from the communication module. Advantageously, the physiological measurement data can be collected and can be further processed in a flexible manner.

Further alternatively, the signal processing module and the communication module can be integrated as a single entity, whereby the measurement data acquisition and processing can be done by said single entity either simultaneously or sequentially.

According to a second aspect of the disclosure, a smart toilet or a smart toilet system is provided that comprises the system according to the first aspect of the disclosure.

In this regard, the smart toilet comprises a toilet seat comprising at least one sensor module and at least one communication module, and a remote device comprising at least one signal processing module. Preferably, the sensor module is arranged on an upper surface of the toilet seat for contacting the user sitting on the toilet seat, especially for facilitating a physical contact and/or a near optical contact with the skin/tissue of the user.

Advantageously, the present disclosure discloses a prospect of embedding the system into static objects, such as the smart toilet, in order to facilitate passive acquisition of physiological parameters in a casual manner, especially to detect bowel movements and/or urination.

Preferably, the remote device is a wireless remote device, e.g. smartphones, computers, personal digital assistants, or external signal processing/analysis devices.

Additionally or alternatively, the remote device may be a cloud-based device, e.g. a cloud server, and the signal processing module may be implemented in the cloud-based device.

Preferably, the at least two source electrodes of the sensor module are arranged on the upper surface of the toilet seat to act as a left leg source electrode and a right leg source electrode. In addition, the at least two receiver electrodes of the sensor module are arranged on the upper surface of the toilet seat to act as a left leg receiver electrode and a right leg receiver electrode. Advantageously, a high sensitivity and accuracy of the measured signals can be ensured.

According to a third aspect of the disclosure, a method for bowel movement detection is provided. The method comprises a step of generating one or more physiological parameter measurement data of a user and a timestamp associated with the one or more physiological parameter measurement data. The method further comprises a step of correlating the one or more physiological parameter measurement data using the associated timestamp in order to detect at least one bowel movement segment and/or an urination process of the user. In this regard, the one or more physiological parameter measurement data comprise at least one electrocardiogram measurement data and at least one bio-impedance measurement data of the user.

According to the invention, the method further comprises a step of comparing the correlation of the one or more physiological parameter measurement data with a reference correlation of the one or more physiological parameter measurement data corresponding to a Bristol stool scale. Moreover, the method comprises a step of determining a firmness level of excrement during the at least one bowel movement segment.

It is to be noted that the method according to the third aspect corresponds to the system according to the first aspect and its implementation forms. Accordingly, the method according to the third aspect achieves the same advantages and effects as the system of the first aspect and its respective implementation forms.

Exemplary embodiments of the disclosure are now further explained with respect to the drawings by way of example only, and not for limitation. In the drawings:
- Fig. 1: shows a first exemplary embodiment of the system according to the first aspect of the disclosure;

- Fig. 2: shows a second exemplary embodiment of the system according to the first aspect of the disclosure;

- Fig. 3: shows a third exemplary embodiment of the system according to the first aspect of the disclosure;

- Fig. 4: shows an exemplary embodiment of the smart toilet according to the second aspect of the disclosure;

- Fig. 5: shows an exemplary graph of measured bio-impedance data over time;
- Fig. 6: shows exemplary graphs of measured heart rate, pulse arrival time, and blood pressure over time;
- Fig. 7: shows an exemplary flow diagram for processing the measured physiological data; and
- Fig. 8: shows an exemplary embodiment of the method according to the third aspect of the invention.

Reference will now be made in detail to the embodiments of the present disclosure, examples of which are illustrated in the accompanying drawings. However, the following embodiments of the present disclosure may be variously modified and the range of the present disclosure is not limited by the following embodiments. Reference signs for similar entities in different embodiments are partially omitted.

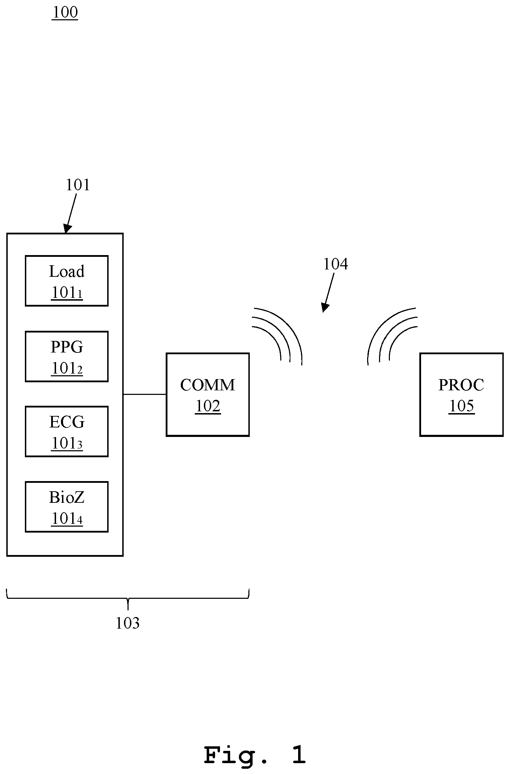

In Fig. 1, a first exemplary embodiment of the system 100 according to the first aspect of the disclosure is illustrated. The system 100 comprises a sensor module or a sensor arrangement or a sensor assembly 101 comprising one or more load sensors or weight sensors 101₁, one or more PPG sensor 101₂, one or more ECG sensor 101₃, and one or more BioZ sensor 101₄. Exemplarily, the sensor module 101 comprises one load sensors 101₁, one PPG sensor 101₂, one ECG sensor 101₃, and one BioZ sensor 101₄.

Although not shown in this example, the sensor module 101 may comprise additional sensors, e.g. one or more temperature sensors such as IR or UV temperature sensors, to generate additional physiological signals. The sensors may be arranged in an array of may be sparsely arranged based on the application of the system 101.

The system 101 further comprises a communication module (COMM) or interface 102 that is operably coupled to the sensor module 101, especially coupled to the sensors 101₁-101₄, and may collect the respective physiological signals from the sensors 101₁-101₄, preferably simultaneously. The communication module 102 may then transmit the collected physiological signals wirelessly over a wireless communication link, e.g. over the communication link 104. The sensor module 101 including the sensors 101₁-101₄, and the communication module 103 may be collectively referred as a sensor system 103.

The system 100 moreover comprises a signal processing module (PROC) 105 that is coupled to the sensor system 103, especially wirelessly. For instance, the signal processing module 105 may receive the physiological signals from the communication module 102 via a wireless communication link, e.g. via the communication link 104. The signal processing module 105 may comprise or be a processor, a microcontroller, a signal processing device, or a combination thereof. The signal processing module 105 may further comprise a memory (not shown) to store the received physiological signals and/or further processed results, e.g. a correlation.

Alternatively, the communication module 102 and the signal processing module 105 can be implemented as a single integrated module, especially within the sensor system 103. In this regard, the integrated module may perform the data acquisition and processing within the system 100 and may transmit the processed results and/or feedback to a remote device (e.g. a smartphone or a personal computer) or a remote system (e.g. a cloud system).

For instance, the sensor module 101 may generate the physiological signals from a user of the system 100, especially while the user having a bowel movement or attempting to have a bowel movement, along with timestamps associated with said physiological signals. Exemplarily the load sensor 101₁ may generate a weight signal with a timestamp, the PPG sensor 101₂ may generate a PPG signal with a timestamp, the ECG sensor 101₃ may generate an ECG signal with a timestamp, and the BioZ sensor 101₄ may generate a BioZ signal with a timestamp.

Accordingly, the communication module 102 may read out the data from the sensors 101₁-101₄, and may transmit the data along with the associated timestamp to the signal processing module 105 via the wireless link 104. The signal processing module 105 may receive the data and the associated timestamp and may correlate the data using the associated timestamp to detect one or more bowel movement segments, especially by detecting a pattern of strain and/or relief from the physiological signals. The signal processing module 105 may further correlate the data using the associated timestamp to detect an urination process, especially by detecting a change in the measured bio-impedance and/or weight.

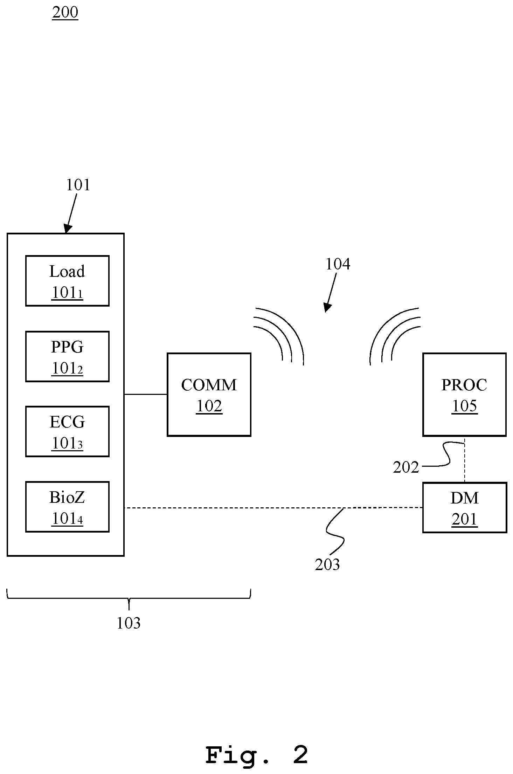

In Fig. 2, a second exemplary embodiment of the system 200 according to the first aspect of the disclosure is illustrated. The system 200 differs from the system 100 in that the system 200 additionally comprises a user detection module (DM) or a user identification module or a user identifier 201.

The user detection module 201 is operably coupled to the signal processing module 105, where the coupling between the user detection module 201 and the signal processing module 105 is exemplarily illustrated by the dashed line 202. The user detection module 201 may be further coupled to the sensor system 103, especially to the sensor module 101, where the coupling is exemplarily illustrated by the dashed line 203.

The sensor module 101, the communication module 102, and the signal processing module 105 of the system 200 correspond to the sensor module 101, the communication module 102, and the signal processing module 105 of the system 100, respectively.

The user detection module 201 may comprise a biometric sensor and may generate a user detection signal 202 corresponding to a user of the system 200. The signal processing module 105 may obtain the user detection signal 202 from the user detection module 201, may generate measurement sessions based on the timestamp associated with the physiological signals, and may assign the measurement sessions to the user based on the user detection signal 202.

Alternatively, the user detection module 201 may detect the user based on the measured physiological signals, thereby generating the user detection signal 202. For instance, the user detection module 201 may detect the user by performing an ECG-based human identification and/or PPG-based human identification and/or weight-based human identification and/or bio-impedance-based human identification. In this regard, the user detection module 201 may extract useful traits or features from the measured physiological signals and may compare with a reference database, e.g. comprising a historical assortment of traits or features corresponding to the physiological parameters.

Although not shown, the system 200 may comprise a sleep/wake-up control module that may receive a further user detection signal 203 from the user detection module 201, and may turn on or turn off the sensor system 103, especially the sensor module 101, based on the user detection signal 203. The sleep/wake-up control module may be implemented within the sensor system 103, or within the signal processing module 105 or within the user detection module 201 or as a separate module. In the case of an externally implemented module, it may generate a remote (e.g. wireless) sleep/wake-up signal to turn on or turn off the sensor module 101.

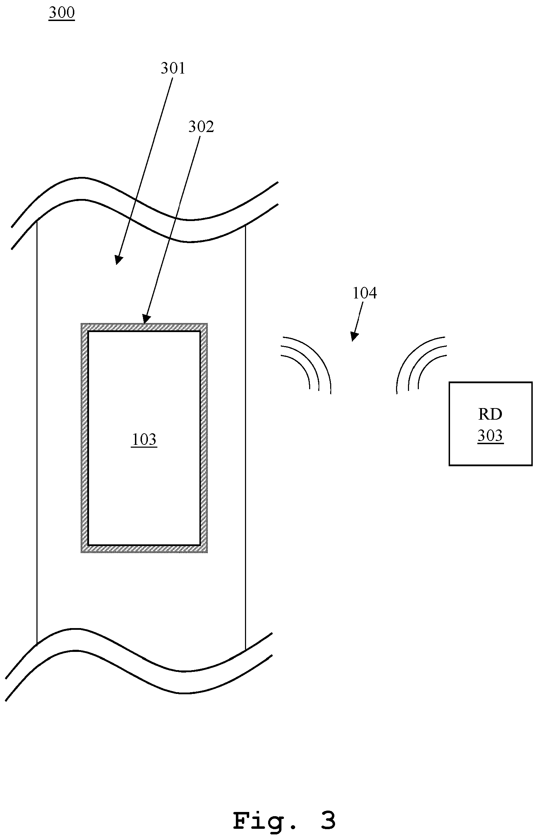

In Fig. 3, a third exemplary embodiment of the system 300 according to the first aspect of the disclosure is illustrated. The system 300 additionally comprises a support member 301 with an accommodation groove 302, whereby the sensor system 103 may be embedded within the support member 301, especially positioned in the accommodation groove 302. The support member 301 can be a part of a seat, a bed, a wristwatch, an armband, and so on. The accommodation groove 302 may be formed such that the sensor system 103, especially the sensor module 101, positioned in the accommodation groove 302 can make near physical and/or optical contact with the skin/tissue of a user of the system 300.

The system 300 additionally comprises a remote device (RD) 303 that comprises the signal processing module 105, and may optionally comprise the user detection module 201. The remote device 303 may comprise or be a wireless remote device, e.g. smartphones, computers, personal digital assistants, or external signal processing/analysis devices. Additionally or alternatively, the remote device 303 may comprise or be a cloud-based device, e.g. a cloud server. The remote device 303 may communicate with the support member 301, especially with the communication module 102 of the sensor system 103, via the communication link 104.

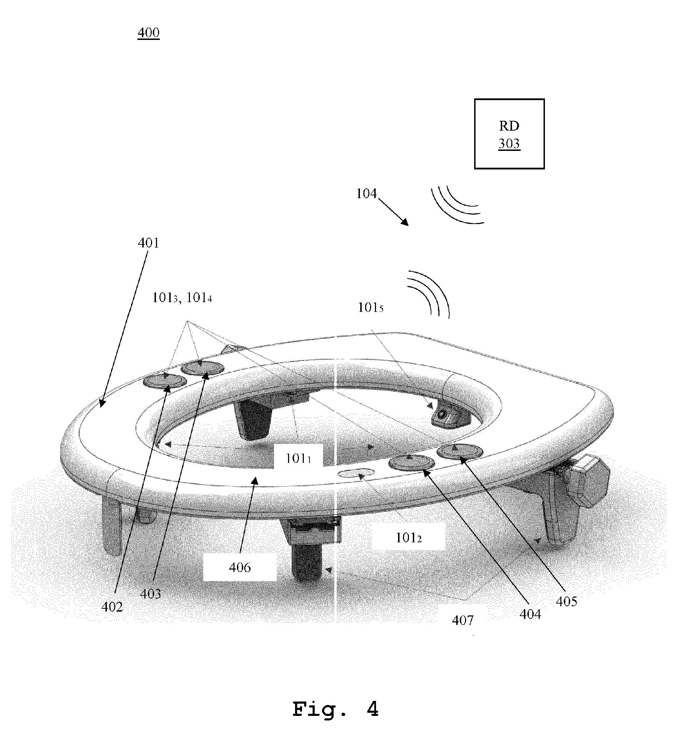

In Fig. 4, an exemplary embodiment of a smart toilet 400 according to the second aspect of the disclosure is illustrated. The smart toilet 400 comprises a toilet seat 401 comprising the sensor system 103, and the remote device 303 comprising the signal processing module 105 and optionally the user detection module 201.

For instance, the sensor module 101 may be sparsely distributed throughout the toilet seat 401 so as to make physical and/or optical contacts to selective parts of the body of the user. Exemplarily, the load sensors 101₁ may be distributed, preferably evenly distributed, along a bottom surface of the toilet seat 401 to facilitate a high precision in weight measurements. The PPG sensors 101₂ may be arranged on an upper surface 406 of the toilet seat 401 for contacting a tissue of the user sitting on the toilet seat 401. The temperature sensors 101₅ may be arranged at a back end of the toilet seat 401.

Moreover, the ECG sensors 101₃ and the BioZ sensors 101₄ may be arranged at the upper surface of the toilet seat 401. In this regard, the upper surface of the toilet seat 401 may be provided with a first source electrode 402 and a second source electrode 404, especially arranged to act as a right leg source electrode and a left leg source electrode, respectively. The upper surface of the toilet seat 401 may be further provided with a first receiver electrode 403 and a second receiver electrode 405, especially arranged to act as a right leg receiver electrode and a left leg receiver electrode, respectively.

The source electrodes 402, 404 and the receiver electrodes 403, 405 may contact the respective body parts of the user to generate ECG and BioZ signals. For instance, the source electrodes 402, 404 and the receiver electrodes 403, 405 may be multiplexed (e.g. frequency division) for parallel acquisition of the ECG signals (low frequency) and the BioZ signals (high frequency). Additionally, the toilet seat 401 may comprise one or more fixation clamps 407 for fixation of the toilet seat 401 on a toilet.

For instance, while having a bowel movement or attempting to have a bowel movement, the user may be detected by the user detection module 201 and the sensors 101₁-101₄ may be turned on accordingly, and the sensors 101₁-101₄ may generate the physiological signals along with timestamps associated to said physiological signals. Exemplarily the load sensors 101₁ may generate weight signals with associated timestamps, the PPG sensors 101₂ may generate PPG signals with associated timestamps, the ECG sensors 101₃ may generate ECG signals with associated timestamp, the BioZ sensors 101₄ may generate BioZ signals with associated timestamps, and the temperature sensor 101₅ may generate temperature signals with associated timestamps.

Accordingly, the communication module 102 may read out the signals from the sensors 101₁-101₅, and may transmit the signals along with the associated timestamps to the remote device 303, especially to the signal processing module 105, via the wireless link 104. The signal processing module 105 may receive the signals and the associated timestamps and may correlate the signals using the associated timestamps to detect one or more bowel movement segments, especially by detecting a pattern of strain and/or relief from the physiological signals.

The signal processing module 105 may also determine the mass or volume of excreta, especially by correlating the weight signals with other physiological signals. For example, if one bowel movement segment is detected and a change in weight signal is detected during said bowel movement segment, the delta or change in weight signal may correspond to the mass of the excrement.

Furthermore, the signal processing module 105 may also determine the amount or volume of urine, especially by correlating the weight signals with other physiological signals, e.g. bio-impedance. For example, if no bowel movement segment is detected and a change in weight signal is detected during this event, the delta or change in weight signal may correspond to the volume of the urine released. As such, in addition to the detection of a bowel movement, the system 400 may detect an urination act or process.

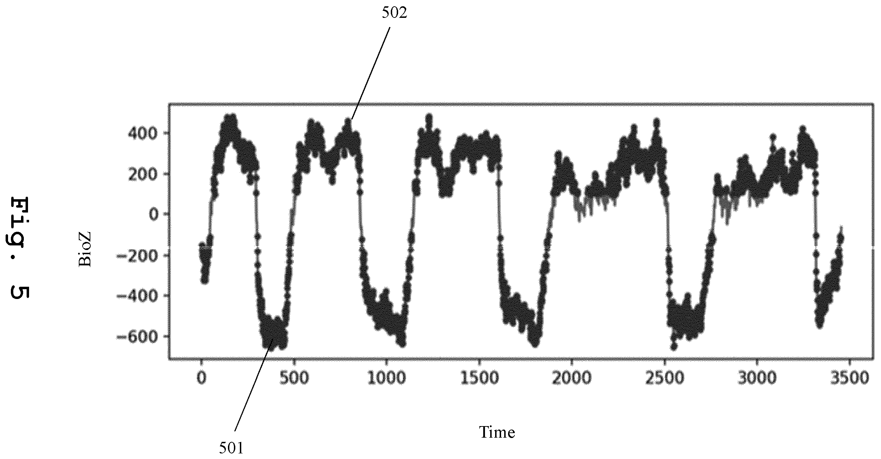

In Fig. 5, an exemplary graph of measured BioZ data over time is illustrated. The horizontal axis denotes time in seconds and the vertical axis denotes BioZ in ohm. The BioZ data may correspond to a BioZ measurement performed by the system 400, especially by passing a high frequency electric current from the right leg source electrode 402, through the adjoined muscle, to the left leg source electrode 404, or vice versa, and by measuring the response via the right leg receiver electrode 403 and the left leg receiver electrode 405, or vice versa.

From the graph, a muscle contraction can be observed at the dips in the graph, e.g. at 501, and a muscle relaxation can be observed at the peaks, e.g. at 502. As such, the BioZ signals over time provide sufficient insight to detect a stress or strain as well as a relief or relaxation, and thereby can be used as a correlation factor for detecting bowel movements.

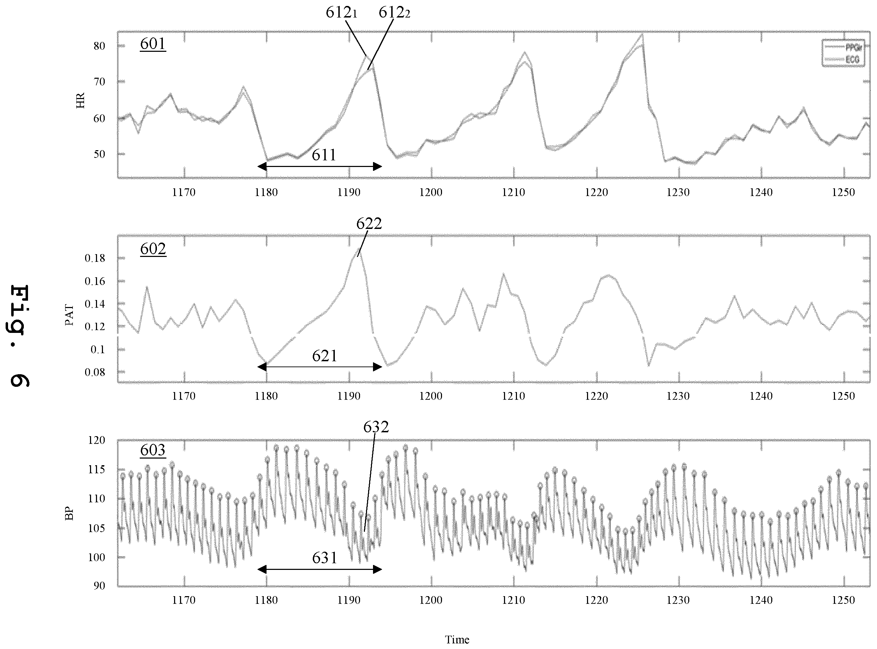

In Fig. 6, exemplary graphs of measured heart rate, pulse arrival time, and blood pressure are illustrated over time. Particularly, the graph 601 shows heart rate (HR) over time, where the horizontal axis denotes the time in seconds and the vertical axis denotes the heart rate in beats per minute, especially measured from the PPG signals 612₁ (e.g. the number of systolic peaks in PPG signals per minute) and also from the ECG signals 612₂ (e.g. intervals between two successive QRS complexes in ECG signals).

Furthermore, the graph 602 shows pulse arrival time (PAT) over time, where the horizontal axis denotes the time in seconds and the vertical axis denotes the pulse arrival time 622, especially obtained by correlating and by extracting temporal features between the ECG signals 612₂ and the PPG signals 612₁.

Moreover, the graph 603 shows blood pressure (BP) over time, where the horizontal axis denotes the time in seconds and the vertical axis denotes blood pressure 632 in mmHg, especially estimated from the pulse arrival time 622 of graph 602. The inverse relationship between the pulse arrival time and the blood pressure, **e.g.** an increment in arterial elastic modulus decreases pulse arrival time, however in turn increases as blood pressure increases, and the technique to estimate blood pressure based on pulse arrival time are well known and therefore will not be described in detail.

Accordingly, a correlation between different physiological parameters or signals can provide sufficient insight to detect stress or strain as well as relaxation, especially during a bowel movement, and therefore one or more bowel movement segments can be detected only by looking at the physiological parameters and/or their correlation.

For instance, during a segment 631 on graph 603, an increment in the blood pressure 632 may indicate a contraction or stress for passing excrement during a bowel movement and a decrement in the blood pressure 632 may indicate a relaxation. Similarly, in graph 602, a segment 621 shows the identical behavior in pulse arrival time 622, albeit inversely due to the inverse relationship between the pulse arrival time 622 and the blood pressure 632. Moreover, a segment 611 also shows the behavior in PPG signals 612₁ and ECG signals 612₂.

For example, a correlation between blood pressure measurements (high blood pressure during bowel movement and low blood pressure during relaxation, which may also be extracted from PPG and/or ECG measurements), and BioZ measurements (dips in BioZ indicating muscle contraction during bowel movement and peaks in BioZ during relaxation) may be effectively utilized to detect a bowel movement, as well as one or more bowel movement segments.

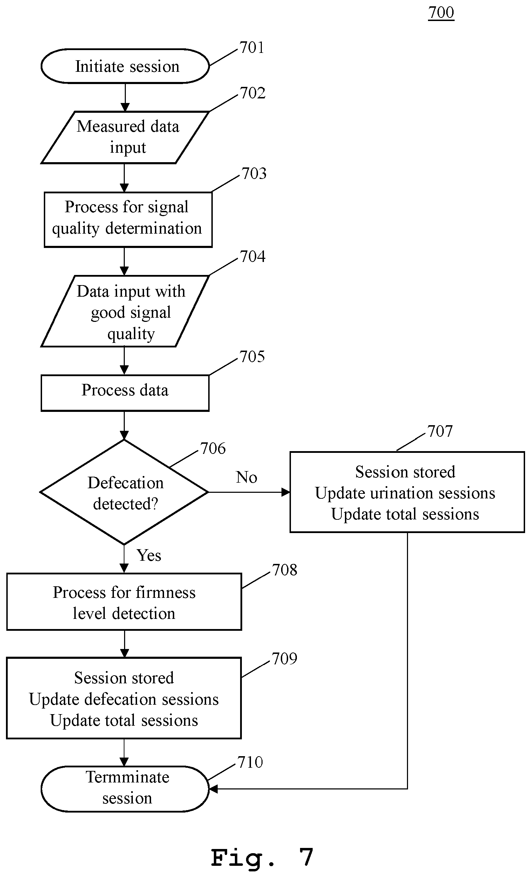

In Fig. 7, an exemplary flow diagram 700 for processing the measured physiological data is illustrated. In a step 701, a measurement session may be initiated. In this regard, the system 100, 200, 300 or the smart toilet 400 may detect a user, e.g. via the user detection module 201, and may turn on the sensors 101₁-101₅ to initiate measurements.

In a further step 702, the measured physiological signals with associated timestamp may be collected, e.g. the communication module 102 may collect the measurements and may transmit to the signal detection module 105.

In a further step 703, a signal quality check may be performed on the measured physiological signals. The quality of the measured signals may be determined based on one or more signal traits, such as but not limited to SNR, peak-to-peak interval, and amplitude, of the measured signal during the physiological signal measurements. Additionally or alternatively, the quality of the measured signal may be determined based on the known signal quality indices for physiological parameters including but not limited to perfusion, kurtosis, and skewness.

In a further step 704, measured physiological signals with good signal quality may be selected or inputted. For example, the communication module 102 may perform the signal quality check and accordingly may transmit only the measured physiological signals with good signal quality to the signal processing module 105. Alternatively, the signal processing module 105 may perform the signal quality check and only consider the measured physiological signals with good signal quality for further processing.

In a further step 705, the measured physiological signals with good signal quality may be further processed to detect a bowel movement or defecation and/or urination. For instance, the signal processing module 105 may correlate the measured physiological signals with good signal quality to identify stress and/or relieve acts or processes corresponding to one or more bowel movement or defecation segments, and may further detect a delta or change in weight during one. Alternatively, the signal processing module 105 may detect a delta or change in weight along with a change in a physiological parameter, e.g. bio-impedance, when no bowel movement or defecation is being detected, thereby detecting an urination act or process.

Upon detecting an urination act or process, i.e. no defecation detected by the system 100, 200, 300 or the smart toilet 400, the session may be stored, e.g. by the signal processing module 105 into the associated memory, and the number of urination sessions as well as the number of total sessions may be updated in a step 707. Afterwards, the session may be terminated in a consecutive step 710.

However, upon detecting defecation, a process for determining a firmness level of excrement may be performed in a step 708. For instance, the signal processing module 105 may perform the process for determining a firmness level of excrement. In this regard, a Bristol stool scale or any other suitable scales known in the art, especially the levels according to the Bristol stool scale, may be categorized using previously collected physiological data and/or reference physiological data and/or correlations with annotated recordings and/or patterns of the physiological signals in order to design a reference database.

For example, a reference correlation and/or patterns of blood pressure changes (i.e. ECG and/or PPG), BioZ changes, and/or weight changes can be pre-defined and be pre-assigned to a stool firmness level of Bristol stool scale, particularly to at least three stool firmness levels including diarrhea, normal, and constipated.

Upon performing a correlation of the measured physiological signals, the reference database may be used for the comparison, and accordingly for the firmness level detection of the excrement during the defecation. Moreover, the reference database, especially the reference correlation, may be generated for the particular user and/or may be assigned to the particular user. Alternatively, the reference database, especially the reference correlation, may be updated and/or amended for different users, e.g. by means of user provided annotations.

In a further step 709, especially after detection defecation and the firmness level of excrement during defecation, the session may be stored, e.g. by the signal processing module 105 into the associated memory, and the number of defecation sessions as well as the number of total sessions may be updated. Afterwards, the session may be terminated in a consecutive step 710.

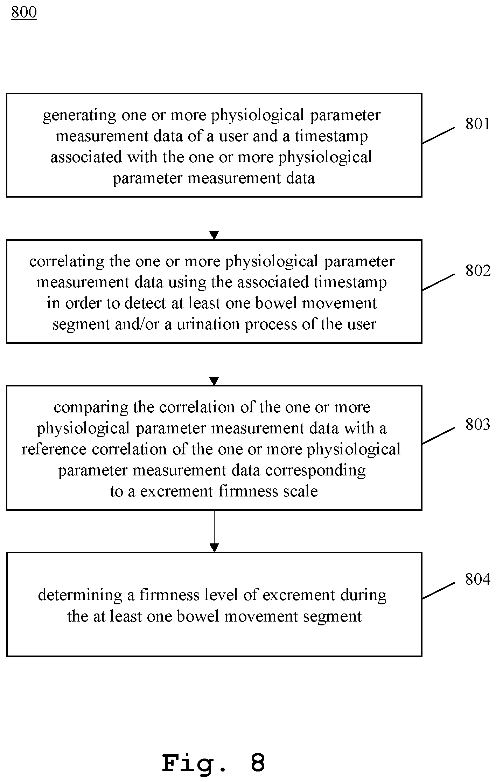

In Fig. 8, an exemplary embodiment of the method 800 according to the third aspect of the invention is illustrated. In a first step 801, one or more physiological parameter measurement data of a user along with a timestamp associated with the one or more physiological parameter measurement data are generated. In a second step 802, the one or more physiological parameter measurement data are correlated using the associated timestamp in order to detect at least one bowel movement segment and/or an urination process of the user. In a third step 803, the correlation of the one or more physiological parameter measurement data is compared with a reference correlation of the one or more physiological parameter measurement data corresponding to an excrement firmness scale. In a fourth step 804, a firmness level of excrement is determined during the at least one bowel movement segment.

## Claims

1. A system (100, 200) for bowel movement detection comprising:
a sensor module (101) configured to generate one or more physiological parameter measurement data of a user and a timestamp associated with the one or more physiological parameter measurement data, and
a signal processing module (105) configured to receive the one or more physiological parameter measurement data and further to correlate the one or more physiological parameter measurement data using the associated timestamp in order to detect at least one bowel movement segment and/or an urination process of the user,
wherein the one or more physiological parameter measurement data comprise at least one electrocardiogram measurement data and at least one bio-impedance measurement data of the user, and **characterized in that** the signal processing module (105) is further configured to compare the correlation of the one or more physiological parameter measurement data with a reference correlation of the one or more physiological parameter measurement data corresponding to a Bristol stool scale, and further to determine a firmness level of excrement during the at least one bowel movement segment.

2. The system according to claim 1,
wherein the sensor module (101) comprises at least two source electrodes (402, 404) configured to excite from at least two different measurement locations on the user, and at least two receiver electrodes (403, 405) configured to respectively measure response from the at least two different measurement locations, especially to generate the at least one electrocardiogram measurement data and/or the at least one bio-impedance measurement data.

3. The system according to claim 2,
wherein the at least two source electrodes (402, 404) and/or the at least two receiver electrodes (403, 405) are multiplexed based on a time division and/or a frequency division multiplexing technique.

4. The system according to any of claims 1 to 3,
wherein the one or more physiological parameter measurement data further comprise at least one weight measurement data, and
wherein the signal processing module (105) is further configured to determine a mass of excrement based on the at least one weight measurement data during the at least one bowel movement segment, and/or
wherein the signal processing module (105) is further configured to determine a volume of urine based on the at least one weight measurement data during the urination process.

5. The system according to any of claims 1 to 4,
wherein the one or more physiological parameter measurement data further comprise at least one photoplethysmography measurement data, and
wherein the sensor module (101) comprises at least one further electrode (406) configured to excite and/or measure response from at least one further measurement location on the user, especially to generate the at least one photoplethysmography measurement data.

6. The system according to any of claims 1 to 5,
wherein the system further comprises a user detection module (201), whereby the signal processing module (105) is configured to obtain a user detection signal (202) comprising at least one user parameter from the user detection module (201), and
wherein the at least one user parameter comprises one or more biometric attributes of the user including fingerprints, facial images, iris, and voice, and/or
wherein the user detection module (201) is configured to detect the user based on the one or more physiological parameter measurement data.

7. The system according to claim 6,
wherein the signal processing module (105) is configured to generate measurement sessions based on the timestamp associated with the one or more physiological parameter measurement data, and further to assign the measurement sessions to the user detection signal (202) and/or to the user.

8. The system according to any of claims 1 to 7,
wherein the signal processing module (105) is configured to assess a signal quality of the one or more physiological parameter measurement data with respect to a pre-defined threshold and further to correlate the one or more physiological parameter measurement data based on the assessed signal quality.

9. The system according to any of claims 1 to 8,
wherein the system further comprises a communication module (102) comprising at least one of a ZigBee module, a Bluetooth module, a Wi-Fi module, a GPRS module, or an NB-IoT module, whereby the communication module (102) is configured to transmit the one or more physiological parameter measurement data wirelessly.

10. The system according to claim 9,
wherein the signal processing module (105) further comprises at least one of a ZigBee module, a Bluetooth module, a Wi-Fi module, a GPRS module, or an NB-IoT module, whereby the signal processing module (105) is further configured to receive the one or more physiological parameter measurement data wirelessly, especially from the communication module.

11. A smart toilet (400) comprising a system according to any of claims 1 to 10 with:
a toilet seat (401) comprising at least one sensor module (101) and at least one communication module (102) according to any of claims 1 to 10, and
a remote device (303) comprising at least one signal processing module (105) according to any of claims 1 to 10, wherein the sensor module (101) is arranged on an upper surface of the toilet seat (401) for contacting the user sitting on the toilet seat.

12. The smart toilet according to claim 11,
wherein the at least two source electrodes (402, 404) of the sensor module (101) are arranged on the upper surface of the toilet seat (401) to act as a left leg source electrode and a right leg source electrode, and the at least two receiver electrodes (403, 405) of the sensor module (101) are arranged on the upper surface of the toilet seat (401) to act as a left leg receiver electrode and a right leg receiver electrode.

13. A computer-implemented method (800) for bowel movement detection comprising:
generating (801) by a sensor module one or more physiological parameter measurement data of a user and a timestamp associated with the one or more physiological parameter measurement data,
correlating (802) the one or more physiological parameter measurement data using the associated timestamp in order to detect at least one bowel movement segment and/or an urination process of the user,
comparing (803) the correlation of the one or more physiological parameter measurement data with a reference correlation of the one or more physiological parameter measurement data corresponding to a Bristol stool scale, and
determining (804) a firmness level of excrement during the at least one bowel movement segment,
wherein the one or more physiological parameter measurement data comprise at least one electrocardiogram measurement data and at least one bio-impedance measurement data of the user.

## Patentansprüche

1. Ein System (100, 200) zur Stuhlgang-Erkennung, aufweisend:
ein Sensormodul (101), das so ausgebildet ist, dass es einen oder mehrere physiologische Parameter-Messdaten eines Benutzers und einen mit dem einen oder den mehreren physiologischen Parameter-Messdaten verbundenen Zeitstempel erzeugt, und
ein Signalverarbeitungsmodul (105), das so ausgebildet ist, dass es die einen oder mehreren physiologischen Parameter-Messdaten empfängt und die einen oder mehreren physiologischen Parameter-Messdaten unter Verwendung des zugehörigen Zeitstempels korreliert, um zumindest einen Stuhlgangsabschnitt und/oder einen Urinierungsvorgang des Benutzers zu erkennen,
wobei die einen oder mehreren physiologischen Parameter-Messdaten Elektrokardiogramm-Messdaten und/oder Bioimpedanz-Messdaten des Benutzers umfassen, und
**dadurch gekennzeichnet, dass**
das Signalverarbeitungsmodul (105) ferner so ausgebildet ist, dass es die Korrelation der einen oder mehreren physiologischen Parameter-Messdaten mit einer einer Bristol-Stuhlskala entsprechenden Referenzkorrelation der einen oder mehreren physiologischen Parameter-Messdaten vergleicht, und, während des zumindest einen Stuhlgangsabschnitts, ferner einen Festigkeitsgrad des Stuhls bestimmt.

2. Das System gemäß Anspruch 1,
wobei das Sensormodul (101) zumindest zwei Sendeelektroden (402, 404) aufweist, die so ausgebildet sind, dass sie an zumindest zwei verschiedenen Messorten am Benutzer anregen, sowie zumindest zwei Empfangselektroden (403, 405), die so ausgebildet sind, dass sie jeweils die Reaktion an den zumindest zwei verschiedenen Messorten messen, insbesondere um die zumindest einen Elektrokardiogramm-Messdaten und/oder die zumindest einen Bioimpedanz-Messdaten zu erzeugen.

3. Das System nach Anspruch 2,
wobei die zumindest zwei Sendeelektroden (402, 404) und/oder die zumindest zwei Empfangselektroden (403, 405) auf der Grundlage einer Zeitmultiplex- und/oder Frequenzmultiplextechnik gemultiplext sind.

4. Das System nach einem der Ansprüche 1 bis 3,
wobei die einen oder mehreren physiologischen Parameter-Messdaten ferner zumindest einen Gewichts-Messwert umfassen, und
wobei das Signalverarbeitungsmodul (105) ferner so ausgebildet ist, dass es, auf der Grundlage des zumindest einen Gewichts-Messwertes, während des zumindest einen Stuhlgangsabschnitts, eine Exkrement-Masse bestimmt, und/oder
wobei das Signalverarbeitungsmodul (105) ferner so ausgebildet ist, dass es, auf der Grundlage des zumindest einen Gewichts-Messwertes, während des Urinierungsvorgangs, ein Urinvolumen bestimmt.

5. Das System nach einem der Ansprüche 1 bis 4,
wobei die einen oder mehreren physiologischen Parameter-Messdaten ferner zumindest einen Photoplethysmographie-Messwert umfassen, und
wobei das Sensormodul (101) zumindest eine weitere Elektrode (406) aufweist, welche so ausgebildet ist, dass sie eine Reaktion an zumindest einem weiteren Messort am Benutzer anregt und/oder misst, insbesondere um den zumindest einen Photoplethysmographie-Messwert zu erzeugen.

6. Das System nach einem der Ansprüche 1 bis 5,
wobei das System ferner ein Benutzererkennungsmodul (201) aufweist, wobei das Signalverarbeitungsmodul (105) so ausgebildet ist, dass es von dem Benutzererkennungsmodul (201) ein Benutzererkennungssignal (202) mit zumindest einem Benutzerparameter erhält, und
wobei der zumindest eine Benutzerparameter ein oder mehrere biometrische Attribute des Benutzers umfasst, einschließlich Fingerabdrücke, Gesichtsbilder, Iris und Stimme, und/oder
wobei das Benutzererkennungsmodul (201) so ausgebildet ist, dass es den Benutzer, auf der Grundlage der einen oder mehreren physiologischen Parameter-Messdaten, erkennt.

7. Das System nach Anspruch 6,
wobei das Signalverarbeitungsmodul (105) so ausgebildet ist, dass es, auf der Grundlage des mit den einen oder mehreren physiologischen Parameter-Messdaten verbundenen Zeitstempels, Mess-Sitzungen erzeugt, und ferner die Mess-Sitzungen dem Benutzererkennungssignal (202) und/oder dem Benutzer zuordnet.

8. Das System nach einem der Ansprüche 1 bis 7,
wobei das Signalverarbeitungsmodul (105) so ausgebildet ist, dass es, in Bezug auf einen vordefinierten Schwellenwert, eine Signalqualität der einen oder mehreren physiologischen Parameter-Messdaten bewertet und ferner die eine oder mehreren physiologischen Parameter-Messdaten auf der Grundlage der bewerteten Signalqualität korreliert.

9. Das System nach einem der Ansprüche 1 bis 8,
wobei das System ferner ein Kommunikationsmodul (102) aufweist, welches zumindest ein ZigBee-Modul und/oder ein Bluetooth-Modul und/oder ein Wi-Fi-Modul und/oder ein GPRS-Modul und/oder ein NB-IoT-Modul umfasst, wobei das Kommunikationsmodul (102) so ausgebildet ist, dass es die einen oder mehreren physiologischen Parameter-Messdaten drahtlos überträgt.

10. Das System nach Anspruch 9,
wobei das Signalverarbeitungsmodul (105) ferner zumindest ein ZigBee-Modul, und/oder ein Bluetooth-Modul und/oder ein Wi-Fi-Modul und/oder ein GPRS-Modul und/oder oder ein NB-IoT-Modul umfasst, wodurch das Signalverarbeitungsmodul (105) ferner so ausgebildet ist, dass es die einen oder mehreren physiologischen Parameter-Messdaten, insbesondere vom Kommunikationsmodul, drahtlos empfängt.

11. Eine intelligente Toilette (400) mit einem System gemäß einem der Ansprüche 1 bis 10, aufweisend:
einen Toilettensitz (401) mit zumindest einem Sensormodul (101) und zumindest einem Kommunikationsmodul (102) gemäß einem der Ansprüche 1 bis 10, und
eine Fernbedienungsvorrichtung (303) mit zumindest einem Signalverarbeitungsmodul (105) gemäß einem der Ansprüche 1 bis 10,
wobei das Sensormodul (101) auf einer Oberseite des Toilettensitzes (401) angeordnet ist, um mit dem auf dem Toilettensitz sitzenden Benutzer in Kontakt zu kommen.

12. Die intelligente Toilette gemäß Anspruch 11,
wobei die zumindest zwei Sendeelektroden (402, 404) des Sensormoduls (101) auf der Oberseite des Toilettensitzes (401) angeordnet sind, um als Sendeelektrode des linken Beins und als Sendeelektrode des rechten Beins zu fungieren, wobei die zumindest zwei Empfangselektrodenelektroden (403, 405) des Sensormoduls (101), um als Sendeelektrode des linken Beins und als Sendeelektrode des rechten Beins zu fungieren, auf der Oberseite des Toilettensitzes (401) angeordnet sind.

13. Ein computergestütztes Verfahren (800) zur Stuhlgang-Erkennung, umfassend:
Erzeugen (801), durch ein Sensormodul, einer oder mehrerer physiologischer Parameter-Messdaten eines Benutzers, und eines mit den einen oder mehreren physiologischen Parameter-Messdaten verbundenen Zeitstempels,
Korrelieren (802) der einen oder mehreren physiologischen Parameter-Messdaten unter Verwendung des zugehörigen Zeitstempels, um zumindest einen Stuhlgangsabschnitt und/oder einen Urinierungsvorgang des Benutzers zu erkennen,
Vergleichen (803) der Korrelation der einen oder mehreren physiologischen Parameter-Messdaten mit einer einer Bristol-Stuhlskala entsprechenden Referenzkorrelation der einen oder mehreren physiologischen Parameter-Messdaten, und
Bestimmen (804) eines Festigkeitsgrades des Stuhls während des zumindest einen Stuhlgangsabschnitts,
wobei die einen oder mehreren physiologischen Parameter-Messdaten zumindest einen Elektrokardiogramm-Messwert und zumindest einen Bioimpedanz-Messwert des Benutzers umfassen.

## Revendications

1. Système (100, 200) de détection de mouvement de l'intestin, comportant:
un module de capteur (101) configuré pour générer une ou plusieurs données de mesure de paramètres physiologiques d'un utilisateur et un horodatage associé à la ou aux données de mesure de paramètres physiologiques, et
un module de traitement de signal (105) configuré pour recevoir la ou les données de mesure de paramètres physiologiques et pour corréler en outre la ou les données de mesure de paramètres physiologiques en utilisant l'horodatage associé afin de détecter au moins un segment de mouvement de l'intestin et/ou un processus de miction de l'utilisateur,
dans lequel la ou les données de mesure de paramètres physiologiques comportent au moins une donnée de mesure d'électrocardiogramme et au moins une donnée de mesure de bio-impédance de l'utilisateur, et
**caractérisé en ce que**
le module de traitement de signal (105) est en outre configuré pour comparer la corrélation de la ou des données de mesure de paramètres physiologiques avec une corrélation de référence de la ou des données de mesure de paramètres physiologiques correspondant à une échelle de selles de Bristol, et pour déterminer en outre un niveau de fermeté des excréments pendant le au moins un segment de mouvement de l'intestin.

2. Système selon la revendication 1,
dans lequel le module de capteur (101) comporte au moins deux électrodes émettrices (402, 404) configurées pour exciter à partir d'au moins deux emplacements de mesure différents sur l'utilisateur, et au moins deux électrodes réceptrices (403, 405) configurées pour mesurer respectivement une réponse provenant des au moins deux emplacements de mesure différents, en particulier pour générer la au moins une donnée de mesure d'électrocardiogramme et/ou la au moins une donnée de mesure de bio-impédance.

3. Système selon la revendication 2,
dans lequel les au moins deux électrodes émettrices (402, 404) et/ou les au moins deux électrodes réceptrices (403, 405) sont multiplexées sur la base d'une technique de multiplexage par répartition dans le temps et/ou par répartition en fréquence.

4. Système selon l'une quelconque des revendications 1 à 3, dans lequel la ou les données de mesure de paramètres physiologiques comportent en outre au moins une donnée de mesure de poids, et
dans lequel le module de traitement de signal (105) est en outre configuré pour déterminer une masse d'excréments sur la base de la au moins une donnée de mesure de poids pendant le au moins un segment de mouvement de l'intestin, et/ou
dans lequel le module de traitement de signal (105) est en outre configuré pour déterminer un volume d'urine sur la base de la au moins une donnée de mesure de poids pendant le processus de miction.

5. Système selon l'une quelconque des revendications 1 à 4, dans lequel la ou les données de mesure de paramètres physiologiques comportent en outre au moins une donnée de mesure de photopléthysmographie, et
dans lequel le module de capteur (101) comporte au moins une électrode supplémentaire (406) configurée pour exciter et/ou mesurer une réponse provenant d'au moins un emplacement de mesure supplémentaire sur l'utilisateur, en particulier pour générer la au moins une donnée de mesure de photopléthysmographie.

6. Système selon l'une quelconque des revendications 1 à 5, dans lequel le système comporte en outre un module de détection d'utilisateur (201), en sorte que le module de traitement de signal (105) est configuré pour obtenir un signal de détection d'utilisateur (202) comportant au moins un paramètre d'utilisateur provenant du module de détection d'utilisateur (201), et
dans lequel le au moins un paramètre d'utilisateur comporte un ou plusieurs attributs biométriques de l'utilisateur incluant des empreintes digitales, des images faciales, des iris et des voix, et/ou
dans lequel le module de détection d'utilisateur (201) est configuré pour détecter l'utilisateur sur la base de la ou des données de mesure de paramètres physiologiques.

7. Système selon la revendication 6,
dans lequel le module de traitement de signal (105) est configuré pour générer des sessions de mesure sur la base de l'horodatage associé à la ou aux données de mesure de paramètres physiologiques, et pour affecter en outre les sessions de mesure au signal de détection d'utilisateur (202) et/ou à l'utilisateur.

8. Système selon l'une quelconque des revendications 1 à 7, dans lequel le module de traitement de signal (105) est configuré pour évaluer une qualité de signal de la ou des données de mesure de paramètres physiologiques par rapport à un seuil prédéfini et pour corréler en outre la ou les données de mesure de paramètres physiologiques sur la base de la qualité de signal évaluée.

9. Système selon l'une quelconque des revendications 1 à 8, dans lequel le système comporte en outre un module de communication (102) comportant au moins un module parmi un module ZigBee, un module Bluetooth, un module Wi-Fi, un module GPRS ou un module NB-IoT, en sorte que le module de communication (102) est configuré pour transmettre la ou les données de mesure de paramètres physiologiques de manière sans fil.

10. Système selon la revendication 9,
dans lequel le module de traitement de signal (105) comporte en outre au moins un module parmi un module ZigBee, un module Bluetooth, un module Wi-Fi, un module GPRS ou un module NB-IoT, en sorte que le module de traitement de signal (105) est en outre configuré pour recevoir la ou les données de mesure de paramètres physiologiques de manière sans fil, en particulier à partir du module de communication.

11. Toilettes intelligentes (400) comportant un système selon l'une quelconque des revendications 1 à 10 avec :
une lunette de toilettes (401) comportant au moins un module de capteur (101) et au moins un module de communication (102) selon l'une quelconque des revendications 1 à 10, et
un dispositif distant (303) comportant au moins un module de traitement de signal (105) selon l'une quelconque des revendications 1 à 10, dans lequel le module de capteur (101) est disposé sur une surface supérieure de la lunette de toilettes (401) pour être en contact avec l'utilisateur assis sur la lunette de toilettes.

12. Toilettes intelligentes selon la revendication 11,
dans lesquelles les au moins deux électrodes émettrices (402, 404) du module de capteur (101) sont disposées sur la surface supérieure de la lunette de toilettes (401) pour agir comme une électrode émettrice de jambe gauche et une électrode émettrice de jambe droite, et les au moins deux électrodes réceptrices (403, 405) du module de capteur (101) sont disposées sur la surface supérieure de la lunette de toilettes (401) pour agir comme une électrode réceptrice de jambe gauche et une électrode réceptrice de jambe droite.

13. Procédé mis en œuvre par ordinateur (800) pour une détection de mouvement de l'intestin, comportant de :
générer (801), par un module de capteur, une ou plusieurs données de mesure de paramètres physiologiques d'un utilisateur et un horodatage associé à la ou aux données de mesure de paramètres physiologiques,
corréler (802) la ou les données de mesure de paramètres physiologiques en utilisant l'horodatage associé afin de détecter au moins un segment de mouvement de l'intestin et/ou un processus de miction de l'utilisateur,
comparer (803) la corrélation de la ou des données de mesure de paramètres physiologiques avec une corrélation de référence de la ou des données de mesure de paramètres physiologiques correspondant à une échelle de selles de Bristol, et
déterminer (804) un niveau de fermeté d'excréments pendant le au moins un segment de mouvement de l'intestin,
dans lequel la ou les données de mesure de paramètres physiologiques comportent au moins une donnée de mesure d'électrocardiogramme et au moins une donnée de mesure de bio-impédance de l'utilisateur.
